(19) Europäisches Patentamt
European Patent Office
Office européen des brevets

(11) Numéro de publication : **0 000 108**
**B1**

(12) # FASCICULE DE BREVET EUROPÉEN

(45) Date de publication du fascicule du brevet : **19.08.81**

(21) Numéro de dépôt : **78400006.9**

(22) Date de dépôt : **01.06.78**

(51) Int. Cl.³ : **C 07 D495/04**, A 61 K 31/445// C07D333/58 ,(C07D495/04, 333/00, 221/00)

(54) **Benzo(b)thiéno-pyridines, leur procédé de préparation et composition thérapeutique les contenant.**

(30) Priorité : **02.06.77 FR 7716878**

(43) Date de publication de la demande : **20.12.78 (Bulletin 78/01)**

(45) Mention de la délivrance du brevet : **19.08.81 Bulletin 81/33**

(84) Etats contractants désignés : **DE NL**

(56) Documents cités :
**FR - A - 2 358 150**
**US - A - 3 752 820**

(73) Titulaire : **OMNIUM FINANCIER AQUITAINE POUR L'HYGIENE ET LA SANTE - SANOFI**
**Tour Aquitaine Cédèx No 4**
**F-92080 Paris La Defense (FR)**

(72) Inventeur : **Frehel, Daniel**
**Résidence La Pléiade 28 Boulevard de la Marne**
**F-31400 Toulouse (FR)**
Inventeur : **Maffrand, Jean Pierre**
**10 Rue Jean Mermoz**
**F-31300 Toulouse (FR)**

(74) Mandataire : **Lavoix, Jean et al**
**c/o Cabinet Lavoix 2, Place D'Estienne D'Orves**
**F-75441 Paris Cedex 09 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

EP 0 000 108 B1

Benzo[b] thiéno-pyridines, leur procédé de préparation et composition thérapeutique les contenant

La présente invention est relative à de nouveaux dérivés de benzo[b] thiénopyridines, à un procédé de préparation de ceux-ci et à leurs applications en médecine humaine et vétérinaire.

Les dérivés de l'invention répondent à la formule

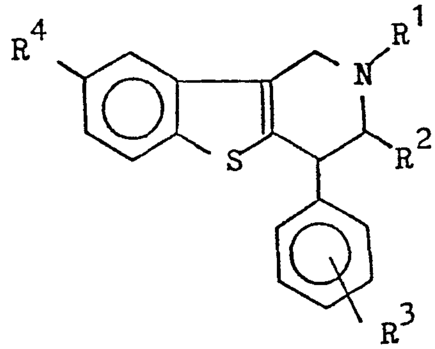

(I)

dans laquelle :

$R^1$ représente l'hydrogène ou un groupe alcoyle inférieur ; phényl-$C_{1-2}$ alcoyle éventuellement substitué sur le noyau phényle par au moins un atome d'halogène ou un groupe hydroxy, nitro, amino, cyano, carboxy, carboxamido, alcoxycarbonyle, alcoyle inférieur, alcoxy inférieur ou trifluorométhyle ; (pyridyl-2)méthyle ; (pyridyl-3)méthyle ou (pyridyl-4)méthyle ;

$R^2$ représente l'hydrogène ou un radical alcoyle inférieur ; et

$R^3$ et $R^4$ représentent chacun l'hydrogène, un atome d'halogène ou un groupe hydroxy, alcoyle inférieur ou alcoxy inférieur.

Par « alcoyle inférieur » ou « alcoxy inférieur » on veut désigner des groupes ayant de 1 à 6 atomes de carbone.

L'invention comprend aussi les sels d'addition des dérivés de formule I avec des acides minéraux ou organiques.

On connaît déjà par le brevet US 3 752 820 des dérivés de benzo[b] thiéno pyridine du type (3,2-c), mais ces dérivés ne portent pas, comme les dérivés de la présente invention, de substituant phényle sur les atomes de carbone du cycle pyridinique.

De plus, ces dérivés sont décrits comme possédant des propriétés tranquillisantes, alors que les dérivés de la présente invention présentent des propriétés sédative et inhibitrice de l'agrégation plaquettaire, ce qui est complètement différent.

Quant à la demande FR 2 358 150 déposée le 13 juillet 1976 mais publiée le 10 février 1978, elle décrit des dérivés de thiéno (2,3-c) et (3,2-c) pyridines et non pas des benzo(b) thiénopyridines.

L'invention a également pour objet un procédé de préparation des composés de formule (I), caractérisé en ce qu'on cyclise des composés de formule (II)

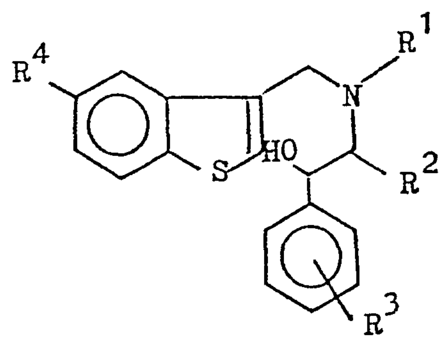

(II)

dans laquelle $R^1$, $R^2$, $R^3$ et $R^4$ ont les significations données pour la formule (I), par chauffage dans l'acide polyphosphorique à des températures comprises entre 60 et 80 °C.

La cyclisation est effectuée de préférence en présence d'un gaz inerte, notamment l'azote.

Les composés (II) utilisés à titre d'intermédiaires sont des composés nouveaux qui peuvent être préparés par des procédés classiques. On peut obtenir, par exemple, les composés de formule (II) comme suit :

On fait réagir un phényl-1 éthanolamine de formule (III) ci-dessous dans laquelle $R^2$ et $R^3$ ont les mêmes significations que ci-dessus, soit avec un formyl-3 benzo(b) thiophène et on fait suivre d'une réduction, soit avec un halogénométhyl-3 benzo(b)thiophène, pour obtenir le composé de formule (IIa) ci-dessous, qui est ensuite le cas échéant condensé avec un halogénure de formule $R^1X$ dans lequel $R^1$ a la

même signification que précédemment et X est un atome d'halogène (chlore, brome ou iode), si on désire que $R^1$ soit autre qu'un atome d'hydrogène.

Le schéma réactionnel est le suivant :

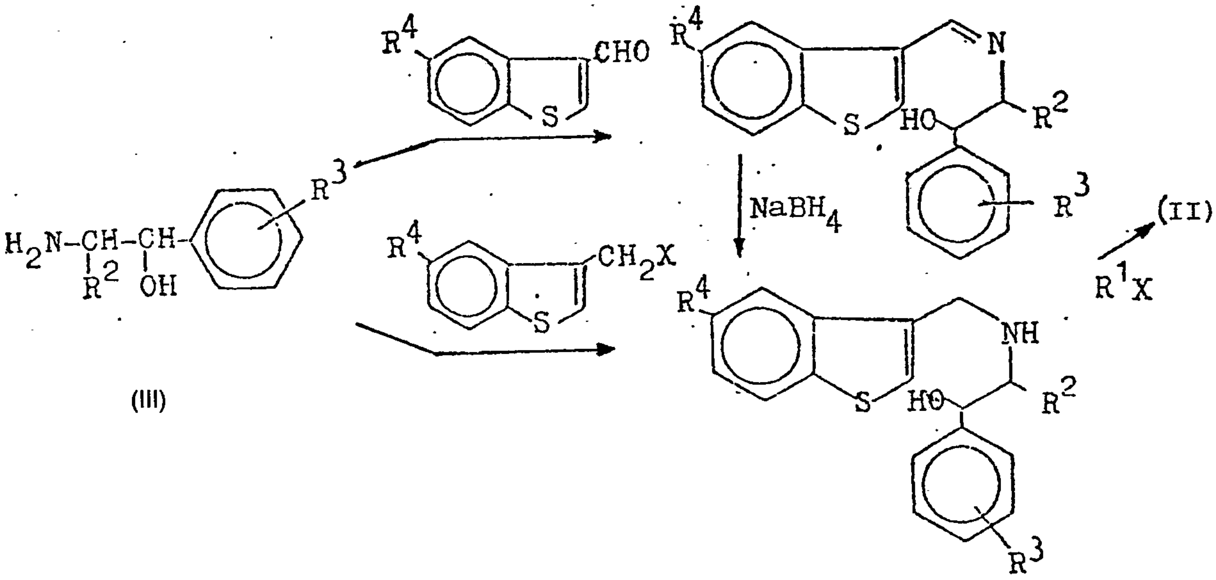


(III)

(IIa) ($R^1$ = H)

Les composés de formules (I) pour lesquels $R^1$ est différent de l'hydrogène peuvent aussi être obtenus par condensation des composés de formule (I) correspondants dans lesquels $R^1$ est de l'hydrogène, avec un composé de formule $R^1X$ dans lequel X est un atome d'halogène. La réaction est effectuée normalement dans un solvant inerte tel que l'éthanol ou le diméthylformamide, en présence d'une base telle qu'un carbonate de métal alcalin, par exemple le carbonate de potassium. Lorsque X est le chlore ou le brome, on peut avantageusement ajouter une quantité catalytique d'un iodure minéral tel que l'iodure de potassium.

Les halogénométhyl-2 ou -3 benzo(b)thiophènes peuvent être préparés selon : S. AVAKIAN, J. MOSS et G.J. MARTIN, J. amer. chem. Soc., 1948, *70,* 3 075 ; N.B. CHAPMAN, K. CLARKE ; B. GORE et S.N. SAWHNEY, J. chem. Soc. (C), 1968, 514 ; N.B. CHAPMAN, K. CLARKE et B. IDDON, J. chem. Soc. (C), 1965, 774. Les formyl-2 et -3 benzo(b)thiophènes peuvent être préparés selon : D.A. SHIRLEY et M.J. DANZIG, J. amer. chem. Soc., 1952, *74,* 2 935 ; K. CLARKE, C.G. HUGUES, A.J. HUMPHRIES et R.M. SCROWSTON, J. chem. Soc. (C), 1970, 1 013 ; M.S. EL SHANTA et R.M. SCROWSTON, J. chem. Soc. (C), 1967, 2 085 ; E. CAMPAIGNE et E.S. NEISS, J. HET. Chem., 1966, *3,* 46.

Les produits de départ de formule (III) sont des produits disponibles dans le commerce ou bien sont décrits dans la littérature.

On prépare les sels d'addition avec des acides minéraux (par exemple l'acide chlorhydrique, sulfurique, etc...) ou organiques (par exemple l'acide méthane sulfonique, maléique, tartrique, citrique, etc...) par les procédés classiques usuels.

Les exemples non limitatifs suivants illustrent l'invention :

Exemple 1

Chloro-8 méthyl-3 phényl-4 tétrahydro-1,2,3,4 benzo(b) thiéno[3,2-c]pyridine ($R^1 = R^3$ = H, $R^2 = CH_3$, $R^4$ = Cl).

a) préparation de l'amino-alcool de formule II

On chauffe à 70 °C, pendant 14 heures, sous atmosphère d'azote, un mélange de 40 g (0,153 mole) de bromométhyl-3 chloro-5 benzo(b)thiophène, 28,7 g (0,153 mole) de chlorhydrate de noréphédrine, 42 g (0,306 mole) de carbonate de potassium sec et 400 $cm^3$ de diméthylformamide. On filtre les sels minéraux et on évapore le solvant à sec sous bon vide. L'huile résiduelle est reprise par du chlorure de méthylène. Les extraits organiques sont lavés à l'eau, séchés sur sulfate de sodium sec et filtrés sur lit de silice. L'évaporation laisse des cristaux que l'on recristallise dans le cyclohexane : cristaux blanchâtres, F = 83 °C, rdt : 71 %.

b) cyclisation de l'amino-alcool

On chauffe à 70 °C pendant 1 heure 30, sous atmosphère d'azote, un mélange agité mécaniquement de 16,7 g (0,05 mole) de l'aminoalcool précédent dans 55 g d'acide polyphosphorique commercial. Après refroidissement le milieu réactionnel est versé sur de la glace, rendu basique avec de l'ammoniaque concentrée et extrait au chlorure de méthylène. Les extraits organiques sont séchés sur sulfate de sodium, filtrés sur lit de silice et évaporés à sec.

Les cristaux obtenus sont recristallisés dans un mélange isopropanol-éthanol : cristaux blancs, F = 174 °C, rdt : 97 %.

Exemple 2

Méthyl-3 phényl-4 tétrahydro-1,2,3,4 benzo(b) thiéno[3,2-c]pyridine ($R^1 = R^3 = R^4 = H$, $R^2 = CH_3$).

a) préparation de l'amino-alcool de formule II

Elle est réalisée à partir du chlorométhyl-3 benzo(b) thiophène et de noréphédine selon le mode opératoire de l'exemple 1 (partie a). Base : cristaux blancs, F = 104 °C (cyclohexane) ; rdt : 67 %.

b) cyclisation de l'amino-alcool

Elle est réalisée selon le mode opératoire décrit à l'exemple 1 (partie b).

Base : cristaux blancs rosés, F = 164 °C (isopropanol) ; rdt : 72,5 %.

Exemple 3

Chloro-8 phényl-4 tétrahydro-1,2,3,4 benzo(b) thiéno[3,2-c]pyridine ($R^1 = R^2 = R^3 = H$, $R^4 = Cl$).

a) préparation de l'amino-alcool de formule II

Elle est réalisée à partir du bromométhyl-3 chloro-5 benzo(b) thiophène et de l'amino-2 phényl-1 éthanol, selon le mode opératoire décrit à l'exemple 1 (partie a).

Base : cristaux blanchâtres, F = 95 °C (cyclohexane) ; rdt : 40 %.

b) cyclisation de l'amino-alcool

Elle est réalisée selon le mode opératoire décrit à l'exemple 1 (partie b).

Méthanesulfonate : cristaux blancs, F = 234 °C (éthanol), rdt : 77 %.

Exemple 4

Phényl-4 tétrahydro-1,2,3,4 benzo(b) thiéno[3,2-c]pyridine ($R^1 = R^2 = R^3 = R^4 = H$)

a) préparation de l'amino-alcool II

Elle est réalisée à partir du chlorométhyl-3 benzo(b) thiophène et de l'amino-2 phényl-1 éthanol, selon le mode opératoire décrit à l'exemple 1 (partie a). Base : cristaux blancs, F = 108 °C (cyclohexane) ; rdt : 34 %

b) cyclisation de l'amino-alcool

Elle est réalisée selon le mode opératoire décrit à l'exemple 1 (partie b).

Méthanesulfonate : cristaux beiges, F = 215 °C (éthanol), rdt : 69 %.

Exemple 5

Chloro-8 diméthyl-2,3 phényl-4 tétrahydro-1,2,3,4 benzo(b) thiéno[3,2-c]pyridine ($R^1 = R^2$ = méthyl ; $R^3 = H$, $R^4 = Cl$)

On effectue la N-méthylation de la chloro-8 méthyl-3 phényl-4 tétrahydro-1,2,3,4 benzo(b) thiéno [3,2-c]pyridine (exemple 1), par condensation avec de l'iodure de méthyle, dans l'éthanol, en présence de carbonate de potassium, ou par réaction de Leuckart (chauffage en présence de formol et d'acide formique).

Chlorhydrate : cristaux blancs, F = 229 °C (isopropanol), rdt : 100 % (réaction de Leuckart).

Exemple 6

Chloro-8 o-chlorobenzyl-2 méthyl-3 phényl-4 tétrahydro-1,2,3,4 benzo(b) thiéno[3,2-c]pyridine ($R^1$ = o.chlorobenzyl ; $R^2$ = méthyl ; $R^3 = H$ ; $R^4 = Cl$)

On chauffe à 70 °C, pendant 12 heures, un mélange de 6 g (0,019 mole) de chloro-8 méthyl-3 phényl-4 tétrahydro-1,2,3,4 benzo(b) thiéno[3,2-c]pyridine (exemple 1), 3,1 g (0,019 mole) de chlorure d'o-chlorobenzyle et 2,6 g (0,019 mole) de carbonate de potassium sec dans 80 $cm^3$ de diméthylformamide. Après refroidissement les sels minéraux sont filtrés et le solvant est évaporé sous pression réduite. Le résidu est repris par de l'eau et extrait au chlorure de méthylène. Les extraits organiques sont lavés à l'eau, séchés sur sulfate de sodium, filtrés sur lit de silice et évaporés à sec. Le résidu est transformé en chlorhydrate que l'on recristallise dans le méthanol : cristaux blancs, F = 175 °C ; rdt : 55 %.

Exemple 7

Benzyl-2 chloro-8 méthyl-3 phényl-4 tétrahydro-1,2,3,4 benzo(b) thiéno[3,2-c]pyridine ($R^1$ = benzyl ;

$R^2$ = méthyl ; $R^3$ = H ; $R^4$ = Cl)

On condense du bromure de benzyle avec la chloro-8 méthyl-3 phényl-4 tétrahydro-1,2,3,4 benzo(b) thiéno[3,2-c]pyridine (exemple 1) selon le procédé décrit à l'exemple 6.

Méthanesulfonate : cristaux blancs, F > 260 °C (acétonitrile), rdt : 63 %.

Exemple 8

Phényl-4 p-tolyl-2 tétrahydro-1,2,3,4 benzo(b) thiéno[3,2-c]pyridine ($R^1$ = p-tolyle ; $R^2$ = $R^3$ = $R^4$ = H)

On condense du bromure de. p-tolyle avec la phényl-4 tétrahydro-1,2,3,4 benzo(b) thiéno[3,2-c] pyridine (exemple 4) selon le procédé à l'exemple 6.

Chlorhydrate : cristaux blancs, F = 200 °C (acétate d'éthyle-méthanol), rdt : 99 %.

Exemple 9

Méthyl-3 m-méthoxybenzyl-2 phényl-4 tétrahydro-1,2,3,4 benzo(b) thiéno[3,2-c]pyridine ($R^1$ = m-méthoxybenzyle : $R^3$ = $R^4$ = H ; $R^2$ = $CH_3$)

On condense du bromure de m-méthoxybenzyle avec la méthyl-3 phényl-4 tétrahydro-1,2,3,4 benzo(b) thiéno[3,2-c]pyridine (exemple 2), selon le procédé décrit à l'exemple 6.

Chlorhydrate : cristaux blancs, F = 160 °C (acétonitrile-méthanol), rdt : 92 %.

Exemple 10

Chloro-8 (triméthoxy-3,4,5 benzyl)-2 méthyl-3 phényl-4 tétrahydro-1,2,3,4 benzo(b) thiéno[3,2-c] pyridine ($R^1$ = triméthoxy-3,4,4 benzyle ; $R^3$ = $R^4$ = H ; $R^2$ = méthyl)

On condense le chlorure de triméthoxy-3,4,5 benzyle avec la chloro-8 méthyl-3 phényl-4 tétrahydro-1,2,3,4 benzo(b) thiéno[3,2-c]pyridine (exemple 1), selon le procédé décrit à l'exemple 6.

Base : cristaux blancs, F = 172 °C, rdt : 78,5 %.

Exemple 11

Chloro-8 o-nitrobenzyl-2 phényl-4 tétrahydro-1,2,3,4 benzo(b) thiéno[3,2-c]pyridine ($R^1$ = o-nitrobenzyl ; $R^2$ = $R^3$ = H ; $R^4$ = Cl)

On condense du chlorure d'o-nitrobenzyle avec la chloro-8 phényl-4 tétrahydro-1,2,3,4 benzo(b) thiéno[3,2-c]pyridine (exemple 3), selon le procédé décrit à l'exemple 6.

Chlorhydrate : cristaux jaunes, F = 150 °C (acétonitrile) ; rdt : 45 %.

Exemple 12

o-cyanobenzyl-2 phényl-4 tétrahydro-1,2,3,4 benzo(b) thiéno[3,2-c]pyridine ($R^1$ = o-cyanobenzyl ; $R^2$ = $R^3$ = $R^4$ = H)

On condense du bromure d'o-cyanobenzyle avec la phényl-4 tétrahydro-1,2,3,4 benzo (b) thiéno [3,2-c] pyridine (exemple 4), selon le procédé de l'exemple 6.

Base : cristaux blanchâtres, F = 143 °C, rdt : 69 %.

Exemple 13

chloro-8 méthyl-3 o-méthoxycarbonylbenzyl-2 phényl-4 tétrahydro-1,2,3,4 benzo(b) thiéno[3,2-c] pyridine ($R^1$ = o-méthoxycarbonylbenzyl ; $R^2$ = $CH_3$ ; $R^3$ = H ; $R^4$ = Cl)

On condense du bromure d'o-méthoxycarbonylbenzyle avec la chloro-8 méthyl-3 phényl-4 tétrahydro-1,2,3,4 benzo(b) thiéno[3,2-c]pyridine (exemple 1), selon le procédé décrit à l'exemple 6.

Chlorhydrate : cristaux blanchâtres, F = 155 °C (acétonitrile), rdt : 86 %.

Exemple 14

o-carboxybenzyl-2 chloro-8 méthyl-3 phényl-4 tétrahydro-1,2,3,4 benzo(b) thiéno[3,2-c]pyridine ($R^1$ = o-carboxybenzyl ; $R^2$ = $CH_3$ : $R^3$ = H ; $R^4$ = Cl.

Ce dérivé est obtenu par hydrolyse basique du composé décrit à l'exemple 13.

Base : cristaux blancs, F = 258 °C (méthanol-diméthylformamide), rdt : 100 %.

Exemple 15

Butyl-2 chloro-8 méthyl-3 phényl-4 tétrahydro-1,2,3,4 benzo(b) thiéno[3,2-c]pyridine ($R^1$ = butyl ; $R^2$ = $CH_3$ ; $R^3$ = H ; $R^4$ = Cl)

On condense du bromure de butyle avec la chloro-8 méthyl-3 phényl-4 tétrahydro-1,2,3,4 benzo(b) thiéno[3,2-c]pyridine (exemple 1), selon le procédé décrit à l'exemple 6.

Méthanesulfonate : cristaux blancs, F = 210 °C (acétonitrile), rdt = 58 %.

Exemple 16

Méthyl-3 phénéthyl-2 phényl-4 tétrahydro-1,2,3,4 benzo(b) thiéno[3,2-c]pyridine ($R^1$ = phénéthyl ; $R^2 = CH_3$ ; $R^3 = R^4 = H$)

On condense du bromure de phénéthyle avec la méthyl-3 phényl-4 tétrahydro-1,2,3,4 benzo(b) thiéno[3,2-c]pyridine (exemple 2), selon le procédé décrit à l'exemple 6.

Chlorhydrate : cristaux blancs, F = 210 °C (isopropanol-méthanol), rdt : 72 %.

Exemple 17

Chloro-8 méthyl-3 phényl-4(pyridyl-3) méthyl-2 tétrahydro-1,2,3,4 benzo(b) thiéno[3,2-c]pyridine ($R^1$ = (pyridyl-3) méthyl ; $R^2 = CH_3$ ; $R^3 = H$ ; $R^4 = Cl$)

On condense du chlorhydrate de chlorométhyl-3 pyridine avec la chloro-8 méthyl-3 phényl-4 tétrahydro-1,2,3,4 benzo(b) thiéno[3,2-c]pyridine (exemple 1) selon le procédé décrit à l'exemple 6.

Dichlorhydrate : cristaux roses, F = 260 °C (méthanoldiméthylformamide), rdt : 69 %.

Exemple 18

Méthyl-3 phényl-4 (pyridyl-2) méthyl-2 tétrahydro-1,2,3,4 benzo(b) thiéno[3,2-c]pyridine ($R^1$ = (pyridyl-2) méthyl ; $R^2 = CH_3$ ; $R^3 = R^4 = H$).

On condense du chlorhydrate de chlorométhyl-2 pyridine avec la méthyl-3 phényl-4 tétrahydro-1,2,3,4 benzo(b) thiéno[3,2-c]pyridine (exemple 2), selon le procédé décrit à l'exemple 6.

Dichlorhydrate : cristaux blanchâtres ; F = 155 °C (acétate d'éthyle-méthanol) ; rdt : 63 %.

Exemple 19

Chloro-8 méthyl-3 phényl-4 (pyridyl-4) méthyl-2-tétrahydro-1,2,3,4 benzo(b) thiéno[3,2-c]pyridine ($R^1$ = (pyridyl-4) méthyl ; $R^2 = CH^3$ ;$R^3 = H$ ; $R^4 = Cl$)

On condense du chlorhydrate de chlorométhyl-4 pyridine avec la chloro-8 méthyl-3 phényl-4 tétrahydro-1,2,3,4 benzo(b) thiéno[3,2-c]pyridine (exemple 1), selon le procédé décrit à l'exemple 6.

Dichlorhydrate : cristaux marron clair, F = 258 °C, rdt : 41 %.

Les résultats des essais toxicologiques et pharmacologiques qui sont rapportés ci-après, ont mis en évidence les intéressantes activités des dérivés de l'invention, notamment inhibitrice de l'agrégation plaquettaire et sédative.

L'invention a donc encore pour objet une composition thérapeutique présentant en particulier des activités inhibitrice de l'agrégation plaquettaire et sédative, caractérisé en ce qu'elle contient, à titre de principe actif, un dérivé de formule (I) ou un sel d'addition avec un acide pharmaceutiquement acceptable de celui-ci, en association avec un véhicule thérapeutiquement administrable.

I - Etude toxicologique

Les composés de l'invention bénéficient d'une excellente tolérance et d'une faible toxicité. Ainsi, la $DL_{50}$/24 h/kg d'animal, déterminée chez la souris selon la méthode de Miller et Tainter, pour la voie orale, est supérieure à 400 mg pour tous les dérivés.

En outre, les essais effectués sur la toxicité aiguë, chronique, sub-chronique et retardée, chez diverses espèces animales, n'ont mis en évidence aucune réaction locale ou générale, aucune perturbation dans les contrôles biologiques régulièrement effectués, aucune anomalie dans les examens microscopiques et macroscopiques chez les animaux sacrifiés et autopsiés en fin d'expérimentation.

II - Etude pharmacologique

1. Action inhibitrice de l'agrégation plaquettaire

Chez des rats de souche Wistar, on effectue un prélèvement sanguin dans la veine jugulaire. A partir de ce sang citraté et après centrifugation, on reconstitue un plasma contenant 600 000 ± 20 000 plaquettes par $mm^3$, qui servira dans toutes les mesures d'agrégation.

a) Mesure de l'agrégation plaquettaire à l'A.D.P.

On place 0,4 ml de plasma dans un tube siliconé pourvu d'une barre aimantée elle-même siliconée. Le tube est introduit dans un agrégomètre couplé à un appareil permettant d'enregistrer les variations de densité optique. Lorsque la transmission de la lumière a atteint une valeur stable, on introduit dans le tube 0,5 ml d'une solution contenant 10 $\mu$ d'A.D.P. (Adénosine-Di Phosphate).

L'agrégation des plaquettes provoque alors une augmentation de la transmission lumineuse suivie

d'une diminution consécutive à la phase de désagrégation.

La variation maximale de densité optique ainsi déterminée caractérise l'intensité de l'agrégation.

b) Mesure de l'agrégation plaquettaire au collagène

La solution d'A.D.P. est remplacée par une solution de collagène (extrait de tendons bovins).

c) Résultats

Différents lots de 20 rats sont utilisés, chaque lot recevant un dérivé à tester par la voie orale, à la dose de 100 mg/kg. Les résultats obtenus au cours de ces 2 essais sont rapportés dans le tableau I suivant qui indique le pourcentage d'inhibition de l'agrégation plaquettaire obtenu, par rapport au témoin, 3 heures après le traitement par le médicament de l'invention, dans le test de l'A.D.P. et au collagène.

TABLEAU I

| Traitement | Pourcentage d'inhibition | |
|---|---|---|
| | A.D.P. | Collagène |
| dérivé de l'Exemple 1 | 62,1 | 91,4 |
| dérivé de l'Exemple 2 | 61,7 | 93,8 |
| dérivé de l'Exemple 3 | 62,9 | 90,5 |
| dérivé de l'Exemple 4 | 61,5 | 90,8 |
| dérivé de l'Exemple 5 | 60,8 | 91,2 |
| dérivé de l'Exemple 6 | 61,2 | 94,4 |
| dérivé de l'Exemple 7 | 63,0 | 95,1 |
| dérivé de l'Exemple 8 | 61,4 | 90,6 |
| dérivé de l'Exemple 9 | 61,8 | 91,2 |
| dérivé de l'Exemple 10 | 60,2 | 94,5 |
| dérivé de l'Exemple 11 | 62,5 | 92,7 |
| dérivé de l'Exemple 12 | 61,3 | 92,2 |
| dérivé de l'Exemple 13 | 62,1 | 95,0 |
| dérivé de l'Exemple 14 | 60,6 | 93,8 |
| dérivé de l'Exemple 15 | 60,1 | 92,6 |
| dérivé de l'Exemple 16 | 62,7 | 93,2 |
| dérivé de l'Exemple 17 | 63,5 | 90,4 |
| dérivé de l'Exemple 18 | 61,3 | 91,7 |
| dérivé de l'Exemple 19 | 62,4 | 91,9 |

2. Action sédative

L'action sédative des composés de l'invention a été étudiée selon plusieurs méthodes.

A) Etude du comportement

Cette étude a été effectuée selon la méthode de SAMUEL IRWIN (Ph. D. animal and clinical Pharmacology Technics in drug evaluation). Les dérivés de l'invention sont administrés par la voie orale à des souris à la dose de 100 mg/Kg. L'étude du comportement des animaux traités, pendant les 4 heures suivantes, ainsi que la mesure des différents paramètres physiologiques, température, vitesse cardiaque et respiratoire, met en évidence la nette action sédative des dérivés de l'invention.

B) Action vis-à-vis des hypnotiques

Les produits à tester sont administrés à des souris par la voie orale à la dose de 100 mg/kg, trente minutes avant l'injection intrapéritonéale d'une solution de 300 mg de chloral dans 20 ml de sérum physiologique. On note le nombre de souris endormies, le temps d'endormissement et la durée du sommeil, par rapport aux souris témoins qui n'ont reçu que l'injection du chloral. On constate que les dérivés de l'invention potentialisent considérablement l'action du chloral, notamment en ce qui concerne la durée du sommeil induit et le nombre de souris endormies.

C) Test de la traction

Ce test consiste à suspendre sur un fil, par les pattes antérieures, des souris qui ont reçu 100 mg du dérivé à tester par la voie orale. On considère que les souris ont subi une action sédative quand elles ne parviennent pas, en trente secondes, à effectuer un rétablissement qui amène au moins une de leurs pattes postérieures sur le fil.

Les animaux sont testés avant l'essai et ceux qui n'arrivent pas à se rétablir dans le temps de trente secondes sont éliminés. On constate au cours des essais que seulement 10 p. 100 des animaux testés réussissent à se rétablir.

D) Test des 4 plaques (Boissier, Simon et Aron, Europ. J. of Pharmacol. 4, 1968, 145-151)

Une souris, placée dans une enceinte contenant 4 plaques électrifiées, reçoit, à chaque passage d'une plaque vers une autre, un stimulus électrique provoquant une fuite désordonnée. Au bout de *n* chocs électriques, la souris ne bouge plus. On considère que le degré de sédation obtenu est proportionnel au nombre *n* de secousses électriques que la souris traitée aura reçu avant qu'elle ne s'immobilise dans un coin.

On détermine ainsi qu'administrés par la voie orale à la dose de 100 mg/Kg, les dérivés de l'invention produisent un pourcentage moyen d'accroissement du nombre de secousses électriques *n* de l'ordre de 60 % après 15 minutes, de 62 % après 30 minutes et de 51 % après quatre vingt dix minutes.

Les résultats de ces études mettent en évidence la bonne tolérance et les intéressantes propriétés inhibitrices de l'agrégation plaquettaire et sédatives des dérivés de l'invention qui les rendent très utiles en médecine humaine et vétérinaire.

Le médicament de l'invention peut être présenté, pour l'administration orale, sous forme de comprimés, comprimés dragéifiés, capsules, gouttes et sirop. Il peut aussi être présenté, pour l'administration rectale, sous forme de suppositoires et pour l'administration parentérale, sous forme de soluté injectable.

Chaque dose unitaire contient avantageusement de 0,010 g à 0,500 g de principe actif, les doses administrabes journellement pouvant varier de 0,010 g à 1,00 g de principe actif selon l'âge du patient et l'affection traitée.

On donnera ci-après, à titre d'exemples non limitatifs, quelques formulations pharmaceutiques du médicament de l'invention.

1) Comprimés

dérivé de l'Ex. 2............0,050 g

excipient : amidon de maïs, stéarate de magnésium, aérosil, talc, amaranthe, tartrazine.

2) Comprimés dragéifiés

dérivé de l'Ex. 6............0,075 g

excipient : talc, amidon de maïs, gomme arabique, gomme laque, sucre, glucose, cire blanche, cire de carnauba, blanc de baleine, lactose, jaune orangé S, oxyde de titane.

3) Capsules

dérivé de l'Ex. 9............0,100 g

excipient : stéarate de magnésium, amidon de maïs, saccharose.

4) Ampoules injectables

dérivé de l'Ex. 14...........0,050 g

excipient : solvant isotonique q.s.p...5 ml

5) Suppositoires

dérivé de l'Ex. 18...........0,100 g

excipient : triglycérides semi-synthétiques

6) Sirop

dérivé de l'Ex. 2............1,00 g

excipient aromatisé q.s.p. 100 ml

Les études toxicologiques et pharmacologiques qui viennent d'être rapportées ont mis en évidence

la bonne tolérance des dérivés de l'invention ainsi que leurs activités inhibitrice de l'agrégation plaquettaire et sédative.

Le médicament de l'invention peut ainsi être administré avec profit, à titre préventif ou curatif, dans le traitement des maladies provoquant une modification pathologique de l'agrégation plaquettaire telles que les maladies provoquant une modification pathologique de l'agrégation plaquettaire telles que les maladies thromboembolliques.

Il peut aussi être administré en tant que sédatif et régulateur du système nerveux, dans l'éréthisme nerveux, la neurotonie, les états d'excitation avec insomnie et les troubles de la dentition.

**Revendications**

1. Composés de formule

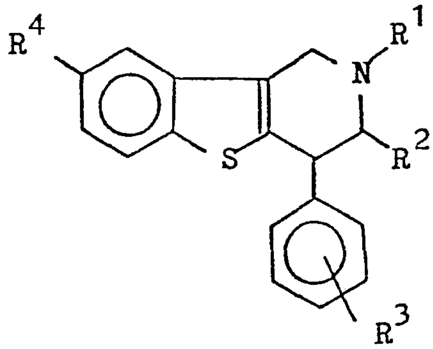

(I)

dans laquelle :

$R^1$ représente l'hydrogène ou un groupe de $C_1$ à $C_6$, phényl-$C_{1-2}$ alcoyle éventuellement substitué sur le noyau phényle par au moins un atome d'halogène ou un groupe hydroxy, nitro, amino, cyano, carboxy, carboxamido, alcoxycarbonyle, alcoyle de $C_1$ à $C_6$, alcoxy de $C_1$ à $C_6$ ou trifluorométhyle ; (pyridyl-2) méthyle ; (pyridyl-3) méthyle ou (pyridyl-4) méthyle ;

$R^2$ représente l'hydrogène ou un radical alcoyle en $C_1$ à $C_6$, et

$R^3$ et $R^4$ représentent chacun l'hydrogène, un atome d'halogène ou un groupe hydroxy, alcoyle en $C_1$ à $C_6$ ou alcoxy en $C_1$ à $C_6$, et leurs sels d'addition avec des acides minéraux ou organiques.

2. Procédé de préparation des composés suivant la revendication 1, caractérisé en ce qu'on cyclise des composés de formule (II)

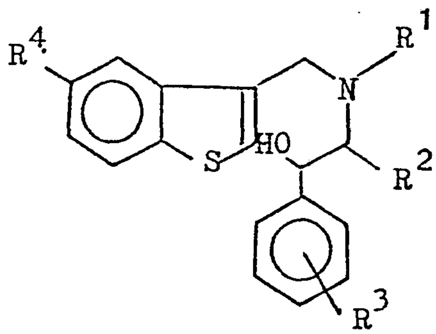

(II)

dans laquelle $R^1$, $R^2$, $R^3$ et $R^4$ sont tels que définis à la revendication 1, par chauffage dans de l'acide polyphosphorique à une température comprise entre 60 et 80 °C.

3. Procédé suivant la revendication 2, caractérisé en ce que la cyclisation est effectuée en présence d'un gaz inerte.

4. Procédé suivant la revendication 2 ou 3, caractérisé en ce que, pour préparer les composés de formule (I) dans lesquels $R^1$ est différent de l'hydrogène, on condense un composé correspondant de formule (I) dans lequel $R^1$ est l'hydrogène avec un composé de formule $R^1X$ dans laquelle $R^1$ est tel que défini à la revendication 1 et X est un atome d'halogène.

5. Procédé suivant la revendication 4, caractérisé en ce que la condensation est effectuée dans un solvant inerte en présence d'une base.

6. Composition thérapeutique ayant notamment une activité inhibitrice de l'agrégation plaquettaire et sédative, caractérisée en ce qu'elle contient comme principe actif, un composé de formule (I) suivant la revendication 1 ou un sel d'addition de celui-ci avec un acide pharmaceutiquement acceptable, associé à un véhicule thérapeutiquement administrable.

7. Composition thérapeutique suivant la revendication 6, caractérisée en ce qu'elle est présentée sous forme de doses unitaires contenant chacune de 0,010 à 0,500 g de principe actif.

**Claims**

1. Compounds having the formula

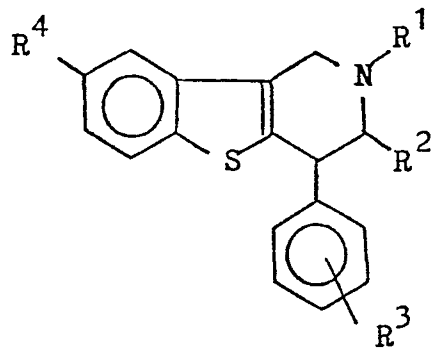

(I)

in which :

$R^1$ represents hydrogen or a $C_{1-6}$ alkyl group ; a phenyl-$C_{1-2}$ alkyl group optionally substituted on the phenyl nucleus with at least a halogen atom or a hydroxy, nitro, amino, cyano, carboxy, carboxamido, alkoxycarbonyl, $C_{1-6}$ alkyl, $C_{1-6}$ alkoxy or trifluoromethyl group ; a (2-pyridyl)-methyl group ; a (3-pyridyl)-methyl group or a (4-pyridyl)-methyl group,

$R^2$ represents hydrogen or a $C_{1-6}$ alkyl radical, and

$R^3$ and $R^4$ represent each hydrogen, a halogen atom or a hydroxy, $C_{1-6}$ alkyl or $C_{1-6}$ alkoxy group, and their addition salts with inorganic or organic acids.

2. Process for the preparation of compounds as defined in claim 1, characterized in that compounds of the formula (II) :

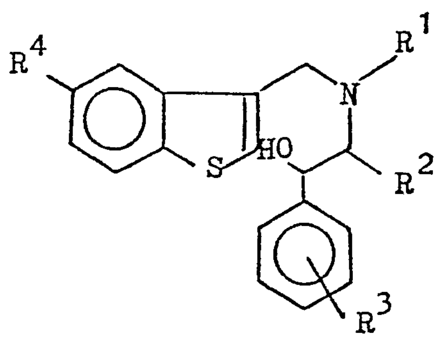

(II)

in which $R^1$, $R^2$, $R^3$ and $R^4$ are as defined in claim 1, are cyclized by heating in polyphosphoric acid at a temperature of 60-80 °C.

3. Process as claimed in claim 2, characterized in that the cyclization is effected in the presence of an inert gas.

4. Process as claimed in claim 2 or 3, characterized in that, to prepare compounds of the formula (I) in which $R^1$ is other than hydrogen, a corresponding compound of the formula (I) in which $R^1$ is hydrogen is condensed with a compound of the formula $R^1X$ in which $R^1$ is as defined in claim 1 and X is a halogen atom.

5. Process as claimed in claim 4, characterized in that the condensation is effected within an inert solvent, in the presence of a base.

6. Therapeutic composition having in particular a sedative and a blood-platelet aggregation inhibiting activity, characterized in that it contains, as active ingredient, a compound of the formula (I) as claimed in claim 1 or a pharmaceutically acceptable acid addition salt thereof, together with a therapeutically administrable carrier.

7. Therapeutic composition as claimed in claim 6, characterized in that it is in unit dosage form, each unit dose containing 0.010-0.500 g active ingredient.

**Ansprüche**

1. Verbindungen der Formel

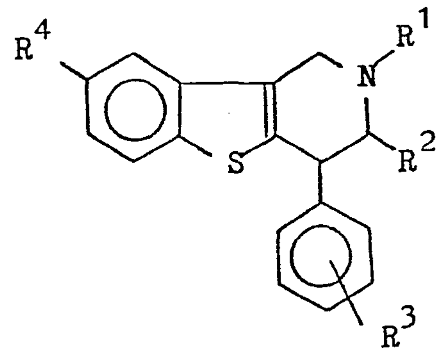

(I)

in der
$R^1$ für Wasserstoff oder eine Alkylgruppe mit 1 bis 6 Kohlenstoffatomen, Phenyl-($C_{1-2}$) alkylgruppe, ggf. am Phenylkern substituiert mit mindestens einem Halogen oder einerHydroxy-, Nitro-, Amino-, Cyan-, Carboxy-, Carboxamido-, Alkoxycarbonyl-, Alkylgruppe mit 1 bis 6 Kohlenstoffatomen, Alkoxygruppe mit 1 bis 6 Kohlenstoffatomen und/oder Trifluormethylgruppe ; 2-Pyridylmethyl- ; 3-Methylpyridyl- und/oder 4-Methylpyridylgruppe steht ; in der
$R^2$ für Wasserstoff und/oder eine Alkylgruppe mit 1 bis 6 Kohlenstoffatomen steht, und in der
$R^3$ und $R^4$ jeweils für Wasserstoff, Halogen oder eine Hydroxygruppe, eine Alkylgruppe mit 1 bis 6 Kohlenstoffatomen und/oder Alkoxygruppe mit 1 bis 6 Kohlenstoffatomen stehen, sowie deren Additionssalze mit organischen oder Mineralsäuren.

2. Verfahren zur Herstellung von Verbindungen gemäß Anspruch 1, *dadurch gekennzeichnet,* daß man Verbindungen der Formel (II)

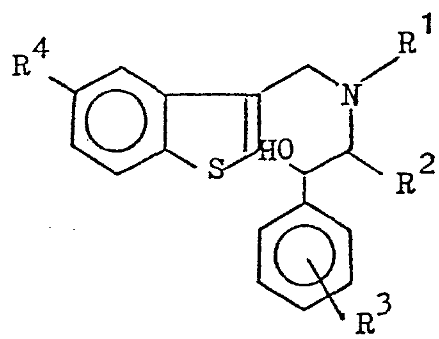

(II)

zyklisiert, wobei $R^1$, $R^2$, $R^3$ und $R^4$ gemäß Anspruch 1 definiert sind, indem man in Polyphosphorsäure bei Temperaturen zwischen 60 und 80 °C erhitzt.

3. Verfahren nach Anspruch 2, *dadurch gekennzeichnet,* daß man in Gegenwart eines inerten Gasen zyklisiert.

4. Verfahren nach Anspruch 2 oder 3, *dadurch gekennzeichnet,* daß man zur Herstellung der Verbindungen gemäß Formel (I), in denen $R^1$ etwas anderes als Wasserstoff ist, eine Verbindung gemäß Formel (I), in der $R^1$ Wasserstoff ist, mit einer Verbindung $R^1X$ kondensiert, wobei $R^1$ gemäß Anspruch 1 definiert und X ein Halogenatom ist.

5. Verfahren nach Anspruch 4, *dadurch gekennzeichnet,* daß man die Kondensation in inertem Lösungsmittel in Gegenwart einer Base durchführt.

6. Therapeutische Zusammensetzung mit vor allem die Aggregation von Blutblättchen inhibierender und sedativer Wirkung, *gekennzeichnet durch* Gehalt an einer Verbindung gemäß Formel (I) gemäß Anspruch 1 oder einem Additionssalz dieser Verbindung mit einer pharmazeutisch verträglichen Säure als Wirkstoffkomponente, zusammen mit einem therapeutisch verabreichbaren neutralen Arzneiaufnahmestoff.

7. Therapeutische Zusammensetzung nach Anspruch 6 in einheitlicher Verabreichungsform mit jeweiligen Gehalt an 0,010 bis 0,500 g der Wirkstoffkomponente.